(19) Europäisches Patentamt
European Patent Office
Office européen des brevets

(11) **EP 4 141 881 A1**

(12) **EUROPEAN PATENT APPLICATION**

(43) Date of publication:
**01.03.2023 Bulletin 2023/09**

(51) International Patent Classification (IPC):
***G16H 20/17*** (2018.01)    ***G16H 20/60*** (2018.01)
***G16H 50/20*** (2018.01)    ***G16H 50/70*** (2018.01)

(21) Application number: **22192768.4**

(22) Date of filing: **30.08.2022**

(52) Cooperative Patent Classification (CPC):
**G16H 20/17; G16H 20/60; G16H 50/20; G16H 50/70**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.08.2021 US 202163239086 P**

(71) Applicant: **Insulet Corporation**
**Acton MA 01720 (US)**

(72) Inventors:
• **LEE, Joon Bok**
  **Acton (US)**
• **ZHENG, Yibin**
  **Hartland (US)**
• **O'CONNOR, Jason**
  **Acton (US)**
• **ANDERSEN, M. Thomas**
  **Highland (US)**

(74) Representative: **Peterreins Schley**
**Patent- und Rechtsanwälte PartG mbB**
**Hermann-Sack-Straße 3**
**80331 München (DE)**

(54) **DEVICES AND METHODS FOR CONTROLLING DRUG DOSAGE DELIVERY FOR AUTOMATICALLY PROVIDING A DRUG TO A PATIENT**

(57)    Disclosed are processes and techniques for a drug delivery system to maintain optimal drug delivery for a patient according to a treatment plan. The disclosed techniques enable a drug delivery system to delivery adjusted drug dosages and/or drug delivery cost function aggressiveness factors modified based on clusters or patterns of patient drug dosages and/or response event probabilities. For example, a controller for operating a drug delivery device may operate to determine a plurality of dosage clusters for a patient based on drug dosage patient information, determine an adjustment profile for each of the dosage clusters, determine a current cluster for a dosage cycle, determine an adjusted dosage for the dosage cycle by applying the adjustment profile to a default dosage, and provide a signal to the drug delivery device to deliver the adjusted dosage to the patient for the dosage cycle. Other embodiments are described.

FIG. 1

EP 4 141 881 A1

**Description**

BACKGROUND

Automated drug delivery systems may operate to provide dosages of a drug based on patient characteristics. For example, an automated drug delivery system may monitor an analyte of the patient and infuse the patient with a dosage of the drug to maintain the analyte within a target value or range. An automatic insulin delivery (AID) system, for instance, may include a closed control loop that seeks to keep a patient blood glucose level close to a target blood glucose level. In a typical AID system, a controller receives a current blood glucose level reading and compares the current blood glucose level to the target blood glucose level and adjusts the basal insulin delivery to attempt to reduce the difference between the target blood glucose level and the current blood glucose level. Although automatic drug delivery systems, such as an AID system, are able to dynamically adjust for variations in the analyte concentration, conventional systems generally become deterministic or semi-deterministic and deliver a same or similar dosage of the drug for different analyte value inputs even if the analyte concentration should require a different dosage. Accordingly, conventional automated drug delivery devices are not able to adequately and efficiently provide dynamic adjustment of drug dosages based on patient activity patterns.

SUMMARY

This Summary is provided to introduce a selection of concepts in a simplified form that are further described below in the Detailed Description. This Summary is not intended to identify key features or essential features of the claimed subject matter, nor is it intended as an aid in determining the scope of the claimed subject matter.
In accordance with various features of the present disclosure, a controller for operating a drug delivery device may include a processor and a memory storing instructions that, when executed by the processor, operate the controller to determine a plurality of dosage clusters for a patient based on drug dosage patient information, determine an adjustment profile for each of the dosage clusters, determine a current cluster for a dosage cycle, determine an adjusted dosage for the dosage cycle by applying the adjustment profile to a default dosage, and provide a signal to the drug delivery device to deliver the adjusted dosage to the patient for the dosage cycle.
In some embodiments of the controller, the drug delivery device may include an automatic insulin delivery (AID) device. In various embodiments of the controller, the drug may include one of a hormone, a protein, a chemotherapy drug, a medication, glucagon, a glucagon-like peptide, or insulin. In some embodiments of the controller, the drug may include insulin.
In various embodiments of the controller, the instructions, when executed by the processor, may operate the controller to determine the plurality of clusters based on a time period. In exemplary embodiments of the controller, the time period may include one of a week, a month, or a year. In some embodiments of the controller, the plurality of clusters may be segmented based on days of the week.
In various embodiments of the controller, the instructions, when executed by the processor, may operate the controller to determine the plurality of clusters based on at least one cluster criterion. In some embodiments of the controller, the at least one cluster criterion may include a dosage deviation in drug dosages between portions of a time period. In various embodiments of the controller, the time period may include a month and the dosage deviation may include a percentage difference in a dose of the drug between consecutive days of the week. In exemplary embodiments of the controller, the percentage difference may include about 10% to about 100%.
In some embodiments of the controller, the instructions, when executed by the processor, may operate the controller to determine the adjustment profile for each of the dosage clusters based on an overall drug dosage mean and a cluster dosage mean. In various embodiments of the controller, the instructions, when executed by the processor, may operate the controller to determine the adjustment profile for each of the dosage clusters based on (a cluster dosage mean)/(an overall drug dosage mean).
In some embodiments of the controller, the drug may include insulin and the default dosage comprising a total daily insulin (TDI) amount.
In accordance with various features of the present disclosure, a method for operating a drug delivery device may include, via a processor of a controller, determining a plurality of dosage clusters for a patient based on drug dosage patient information, determining an adjustment profile for each of the dosage clusters, determining a current cluster for a dosage cycle, determining an adjusted dosage for the dosage cycle by applying the adjustment profile to a default dosage, and providing a signal to the drug delivery device to deliver the adjusted dosage to the patient for the dosage cycle.
In some embodiments of the method, the drug delivery device may include an automatic insulin delivery (AID) device. In various embodiments of the method, the drug may include one of a hormone, a protein, a chemotherapy drug, a medication, glucagon, a glucagon-like peptide, or insulin. In some embodiments of the method, the drug may include insulin.

In some embodiments of the method, the method may include determining the plurality of clusters based on a time period. In various embodiments of the method, the time period may include one of a week, a month, or a year. In exemplary embodiments of the method, the plurality of clusters may be segmented based on days of the week.

In some embodiments of the method, the method may include determining the plurality of clusters based on at least one cluster criterion. In various embodiments of the method, the at least one cluster criterion comprising a dosage deviation in drug dosages between portions of a time period. In some embodiments of the method, the time period may include a month and the at least one and the dosage deviation comprising a percentage difference in a dose of the drug between consecutive days of the week. In various embodiments of the method, the percentage difference may include about 10% to about 100%.

In exemplary embodiments of the method, the method may include determining the adjustment profile for each of the dosage clusters based on an overall drug dosage mean and a cluster dosage mean. In some embodiments of the method, the method may include determining the adjustment profile for each of the dosage clusters based (a cluster dosage mean)/(an overall drug dosage mean). In various embodiments of the method, the drug may include insulin and the default dosage comprising a total daily insulin (TDI) amount.

In accordance with various features of the present disclosure, a method for operating a drug delivery device to deliver a drug to a patient may include, via a processor of a controller, determining a response event probability for at least one response event for at least one historical time period, determining an aggressiveness factor based on the response event probability, determining a drug dosage for the drug based on the aggressiveness factor applied in a drug delivery cost function for a current time period associated with the at least one historical time period, and delivering the drug dosage of the drug to the patient.

In some embodiments of the method, the drug delivery device may include an automatic insulin delivery (AID) device. In some embodiments of the method, the drug may include one of a hormone, a protein, a chemotherapy drug, a medication, glucagon, a glucagon-like peptide, or insulin. In some embodiments of the method, the drug may include insulin.

In some embodiments of the method, the response event may include at least one of a hypoglycemic event or a hyperglycemic event. In some embodiments of the method, the at least one time period may include a delivery cycle.

In some embodiments of the method, the aggressiveness factor may be determined based on the following estimated aggressiveness factor R':

$$R'(i) = 9000 \cdot \left(1 + 0.2 \cdot P_{hypo}(i)\right)^2 \cdot \left(1 - 0.2 \cdot P_{hyper}(i)\right)^2,$$

where $P_{hypo}$ is the probability of a hypoglycemic response event and $P_{hyper}$ is the probability of a hyperglycemic response event.

In some embodiments of the method, the aggressiveness factor (R) may be determined according to the following:

$$R(i) = f(R(i-1), R(i)').$$

In accordance with various features of the present disclosure, a controller for operating a drug delivery device may include a processor and a memory storing instructions that, when executed by the processor, operate the controller to determine a response event probability for at least one response event for at least one historical time period, determine an aggressiveness factor based on the response event probability, determine a drug dosage for the drug based on the aggressiveness factor applied in a drug delivery cost function for a current time period associated with the at least one historical time period, and deliver the drug dosage of the drug to the patient.

In some embodiments of the controller, the drug delivery device may include an automatic insulin delivery (AID) device. In various embodiments of the controller, the drug may include one of a hormone, a protein, a chemotherapy drug, a medication, glucagon, a glucagon-like peptide, or insulin. In exemplary embodiments of the controller, the drug may include insulin.

In some embodiments of the controller, the response event may include at least one of a hypoglycemic event or a hyperglycemic event. In various embodiments of the controller, the at least one time period may include a delivery cycle.

In some embodiments of the controller, the aggressiveness factor may be determined based on the following estimated aggressiveness factor R':

$$R'(i) = 9000 \cdot \left(1 + 0.2 \cdot P_{hypo}(i)\right)^2 \cdot \left(1 - 0.2 \cdot P_{hyper}(i)\right)^2,$$

where $P_{hypo}$ is the probability of a hypoglycemic response event and $P_{hyper}$ is the probability of a hyperglycemic response event.

In various embodiments of the controller, the aggressiveness factor (R) may be determined according to the following:

$$R(i) = f(R(i-1), R(i)').$$

BRIEF DESCRIPTION OF THE SEVERAL VIEWS OF THE DRAWINGS

FIG. 1 illustrates a first exemplary embodiment of an operating environment in accordance with the present disclosure;
FIG. 2 illustrates patient profile information in accordance with the present disclosure;
FIG. 3 illustrates patient adjustment profile information for a profile-based dosage adjustment process in accordance with the present disclosure;
FIG. 4 illustrates a first logic flow in accordance with the present disclosure;
FIG. 5 illustrates a second logic flow in accordance with the present disclosure;
FIG. 6 illustrates patient profile information for a profile-based aggressiveness adjustment process in accordance with the present disclosure;
FIG. 7 illustrates a third logic flow in accordance with the present disclosure; and
FIG. 8 illustrates a functional block diagram of a system in accordance with the present disclosure.

The drawings are not necessarily to scale. The drawings are merely representations, not intended to portray specific parameters of the disclosure. The drawings are intended to depict example embodiments of the disclosure, and therefore should not be considered as limiting in scope. In the drawings, like numbering represents like elements.

DETAILED DESCRIPTION

As described below, a drug delivery system may include a number of components, such as a drug delivery device, a controller, and an analyte sensor. The drug delivery device may be controlled and operate based on signals containing information received from the analyte sensor. Control may be based on an application executing on the drug delivery device, the controller, the analyte sensor, or may be distributed between two or more of the drug delivery device, the controller, or the analyte sensor. For example, the drug delivery device may be configured to deliver a drug at a dosage or frequency based on a measured analyte concentration associated with a patient.

An example of an automatic analyte control application or logic circuit may be specific to a particular type of analyte. Non-limiting examples of analytes may include blood glucose, a hormone, a protein, a medication, ketone, alcohol level, and the like. A control application specific to controlling a patient's blood glucose may be an Automatic Glucose Control (AGC) application that may rely on a blood glucose measurement value and other information that may be provided by a continuous glucose monitor (CGM). "Automatic glucose control" may refer to an algorithm or computer application that is operable to use blood glucose measurements to determine amounts of a drug to be delivered to a user in order to maintain the user's measured blood glucose within a set range of blood glucose measurement values. "Blood glucose measurement value" may refer to a numerical value representative of an amount of glucose in a user's blood that has the units of milligrams per deciliter (mg/dL). The other information provided by the CGM may be included as part of, or separately from, the blood glucose measurement value. The AGC may be operable to estimate and store the estimated values of CGM based upon various parameters within the drug delivery system. Such future trend information may be useful in case of missing CGM values to estimate suggested insulin for a short duration.

Although insulin delivery devices, insulin, blood glucose, AGC applications, and CGM devices are used in examples in the present disclosure, embodiments are not so limited as these are provided for illustrative purposes. Embodiments may operate with various types of fluid or drug delivery devices, drugs or other fluids, analytes, automated drug delivery applications, and/or analyte sensors in accordance with the teachings of the present disclosure.

In some embodiments, a drug delivery system may perform a profile-based dosage adjustment process to determine a dosage of a drug for delivery to a patient based on one or more patient characteristics. In various embodiments, a patient characteristic may be or may include a dosage of the drug provided during a time segment using an analyte-based dosage process (for instance, the dosage of the drug is based on a measured analyte to maintain the analyte within a target range). Non-limiting examples of a time segment may include an hour, a day, a weekday, four or five weekdays (e.g., Monday, Tuesday, Wednesday, Thursday, and/or Friday), two or three weekend days (e.g., Friday, Saturday, and/or Sunday), one weekend day, a week, a month, a year, a holiday, a season, an activity (for instance, working, at school, exercising, and/or the like) and/or a portion or combination thereof. For example, a patient may require different levels of the drug during different time segments. In one non-limiting example, a patient may require more insulin during winter months as compared with summer months due to being more sedentary and/or having a different diet during winter compared with during summer. In another non-limiting example, a patient may require a different amount of insulin during weekdays (for instance, when at work or school) compared with weekends (for instance, due to increased carbohydrate intake).

Accordingly, some embodiments may process historical dosage amounts based on discrete time segments to determine dosage clusters (for instance, a weekend day cluster, a weekday cluster, and/or the like). An automatic dosage delivery system may administer the drug based, at least in part, on the dosage clusters.

For example, AID algorithms are designed to dynamically adjust for variations in the user's glucose concentration and increase or decrease insulin delivery. However, for a given set of input conditions, deterministic systems will always deliver the same outputs, not considering patterns that are not reflected in current blood glucose data (e.g., recurrent increases or decreases in blood glucose values on a weekly basis due to consistent user behavior on a particular day or time of day). Therefore, if the input values remain the same, an AID algorithm will always deliver the same amount of insulin for a given glucose excursion even if the patient may require additional or reduced insulin. Accordingly, conventional AID systems (and other drug delivery systems) may be improved to dynamically adjust for additional patterns and augment the AID algorithm with modified inputs. For example, for a typical office worker whose living patterns may significantly differ based on large overarching patterns, such as sedate activity levels during weekdays or business hours, versus higher-activity levels during weekends (or vice versa). Some embodiments may provide dosing processes that use clustering methods associated with long term changes in the patient's dosage (for instance, TDI for insulin) and utilizing this pattern to improve the input drug delivery values to an automated drug delivery process.

In some embodiments, a drug delivery system may perform a profile-based aggressiveness adjustment process to determine a dosage of a drug for delivery to a patient based on one or more patient characteristics. In various embodiments, a profile-based aggressiveness adjustment process may operate to adjust the aggressiveness (for instance, via an aggressiveness factor) of a cost function for determining drug delivery dosages for a drug delivery device based on historical data associated with a user. In some embodiments, the historical data may be or may include data indicative of a user reaction or response event to a dosage of a medicament provided by the drug delivery device. In general, a response event may include an event, reaction, response, and/or the like indicating a patient reaction to a drug and/or a particular concentration of a drug. For example, a response event may include a blood analyte concentration being over a threshold, a blood analyte concentration being under a threshold, a concentration of the delivered drug in the blood being over a threshold, a concentration of the delivered drug in the blood being under a threshold, and/or the like. In general, a response event may include any measurable patient reaction to a delivered drug. For an insulin (or other glucose control drug, including, without limitation, glucagon or a glucagon-like peptide) drug delivery device, a response event may be a hypoglycemic event, a hyperglycemic event, a normal blood glucose range event, and/or the like.

In general, the aggressiveness of a drug delivery device may be or may include a tuning parameter, gain, error term, or other factor associated with device output (i.e., drug delivery dosage) in response to device input (i.e., measured analyte that controls the drug delivery dosage amount). For a blood glucose control system, aggressiveness may be associated with how quickly a system tries to reduce the user's blood glucose to be within a blood glucose measurement range considered normal for the use. The aggressiveness factor (often denoted as "R" in automated insulin (or other glucose control) delivery systems) determines the speed of response of the AID algorithm to changes in glucose concentration. A relatively conservative value of R results in a controller that is slow to adjust insulin infusion amounts (relative to basal) in response to changes in glucose; a relatively aggressive value of R results in a controller that is quick to respond to changes in glucose. The aggressiveness factor may be used in a cost function is usable in determining an amount of a dosage of insulin for the user.

In some embodiments, a profile-based aggressiveness adjustment process for a diabetes treatment regimen, the historical data may include previous glucose concentrations. In some embodiments, the previous glucose concentrations may be used to determine a probability or risk of a patient hypoglycemic event or a hyperglycemic event. In this manner, a drug delivery system may operate to reference past outcomes of the user's glucose concentrations (for instance, each cycle or other interval) to assess the probability that the user may experience hypo- or hyperglycemic risk. For example, even when the user's glucose concentration is similar, the drug delivery system may deliver less/more aggressively if the user has always had hypoglycemic/hyperglycemic risk during a certain period of time, compared to periods when the user did not have hypoglycemic/ hyperglycemic risk.

Accordingly, some embodiments may provide dosing processes that use time and/or event clustering methods to determine an aggressiveness of a drug delivery process. The profile-based aggressiveness adjustment process may operate to protect patients from adverse or otherwise unwanted response events based on time and/or event-based historical information associated with the patient.

Therefore, drug delivery systems and methods for operating drug delivery devices according to some embodiments may provide multiple technological advantages over conventional systems and methods. In one non-limiting technological advantage, systems may be configured to provide analyte-based drug delivery control based on dosage clusters. In some embodiments, analyte-based drug delivery control may include control of dosage amounts and/or dosage aggressiveness based on dosage clusters or other historical patient information. Conventional devices were only capable of proceeding with a drug delivery approach based on previous dosage amounts without accounting for time-based dosage clusters or patterns, event-based clusters or patterns, micropatterns, and/or the like. As a result, conventional drug delivery systems typically become deterministic and are not able to provide accurate dosage control to patients based on real-world patient activity patterns. In addition, conventional drug delivery systems do not have the functionality to protect patients from adverse response events based on probabilities or risks associated with time or event cluster information (which, in some embodiments, may be addressed via controlling dosage aggressiveness based on historical information, clusters, patterns, and/or the like).

The non-limiting technological advantages may include improvements in computing technology and/or a technical field. For instance, systems and methods according to some embodiments may improve conventional logic devices, controllers, computing devices, and/or other computing technology for controlling drug delivery systems by providing computing technology capable of analyte-based drug delivery control with dosage adjustments and/or aggressiveness adjustments based on patient dosage clusters. In addition, systems and methods according to some embodiments may improve the field of automated drug delivery, including, for example, wearable insulin drug delivery devices and associated processes for controlling these devices, drug therapy, diabetes therapy, and/or the like. Furthermore, the methods according to some embodiments may be applied to the practical applications of controlling a drug delivery device, providing a dosage of a drug to a patient, drug therapy, and/or diabetes therapy. Embodiments are not limited in this context. Additional technological advantages, practical applications, and improvements to computing technology and/or technical fields would be known to a person of skill in the art in view of the teachings of the present disclosure.

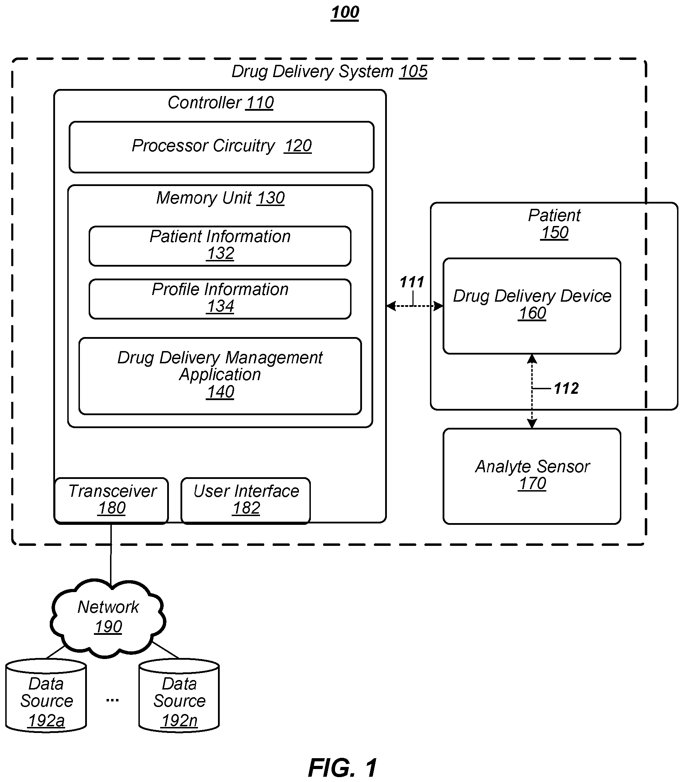

**FIG. 1** illustrates features of the subject matter in accordance with an embodiment of a drug delivery system 105 of an operating environment 100. "Drug delivery system" may refer to a drug delivery device and an analyte sensor, such as a continuous glucose monitor (CGM), and, in some configurations, a controller. "Drug delivery device" may refer to a device configured to administer a liquid drug to a user. A drug delivery device may be the same or substantially similar to the OmniPod® device or system provided by Insulet Corporation of Acton, Massachusetts, United States, for example, as described in United States Patent Nos. 7,303,549; 7,137,964; and/or 6,740,059, each of which is incorporated herein by reference in its entirety.

The drug delivery device may respond to command signals received from a controller, which may be a separate device. Alternatively, the drug delivery device may be configured with a processor that is not separate from the drug delivery device but is operable to perform functions the same as a remote controller. "Controller" may refer to a device having a processor that is configured to control operation of a drug delivery device and transmit and receive information related to measured blood glucose of a user associated with the drug delivery device and the controller. The functions performed by the controller may be executed by a processor integral to the drug delivery device. "Continuous glucose monitor" may refer to a wearable device that may at least include a sensor configured to measure blood glucose of a wearer, a transmitter to transmit blood glucose measurement values measured by the sensor, and logic circuitry that controls the sensor and the transmitter.

Drug delivery system 105 may include a logic device or controller 110, drug delivery device 160, and an analyte sensor 170. Controller 110 may communicate with drug delivery device 160 via communication link 111 and drug delivery device 160 may communicate with the analyte sensor 170 via a communication link 112. "Communication link" may refer to a signal pathway between a first device and a second device. As described below, information may be exchanged between the first device and the second device where both devices may transmit and receive information or other signals.

In some embodiments, controller 110 may be a smart phone, PDM, or other mobile computing form factor in wired or wireless communication with drug delivery device 160. For example, controller device 110 and drug delivery device 160 may communicate via various wireless protocols, including, without limitation, Wi-Fi (i.e., IEEE 802.11), radio frequency (RF), Bluetooth®, Zigbee™, near field communication (NFC), Medical Implantable Communications Service (MICS), and/or the like. In another example, controller 10 and adjustment compliance device may communicate via various wired protocols, including, without limitation, universal serial bus (USB), Lightning, serial, and/or the like.

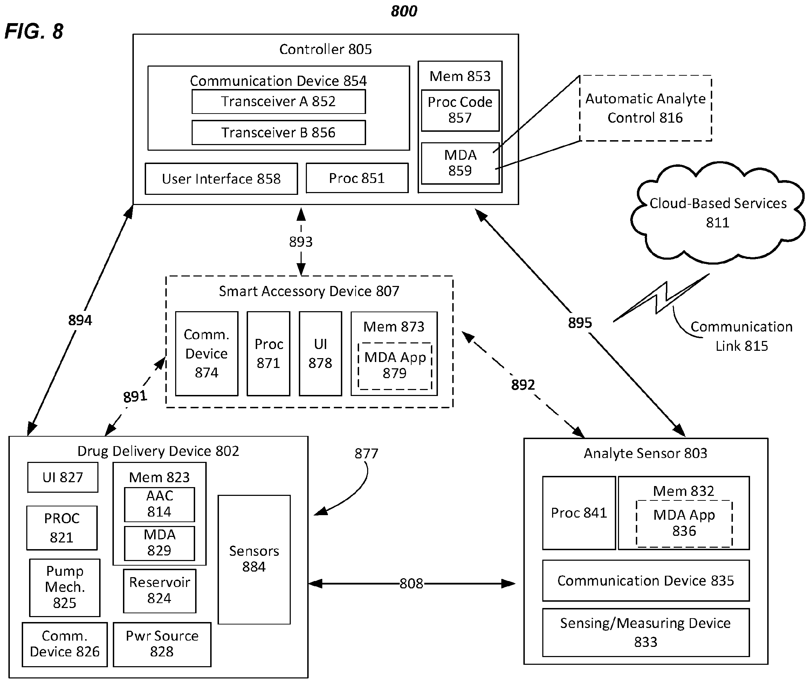

Although controller 110 (and components thereof), drug delivery device 160, and analyte sensor 170 are depicted as separate devices, embodiments are not so limited. For example, in some embodiments, controller 110, drug delivery device 160, and/or analyte sensor 170 may be a single device. In another example, some or all of the components of controller 110 may be included in drug delivery device 160. For example, drug delivery device 160 may include processor circuitry 120, memory unit 130, and/or the like. In some embodiments, each of controller 110 and drug delivery device 160 may include a separate processor circuitry 120, memory unit 130, and/or the like capable of facilitating automatic analyte control and/or device initialization processes according to some embodiments, either individually or in operative combination. Embodiments are not limited in this context (see, for example, FIG. 8)

Drug delivery device 160 may include at least one housing and a number of components to facilitate automated delivery of a drug to patient 150. For example, drug delivery device 160 may include a reservoir for storing the drug, a needle and/or cannula for delivering the drug into the body of the patient, and a pump for transferring the drug from the reservoir, through the needle or cannula, and into the body of the patient. In some embodiments, the drug may be or may include insulin, glucagon like peptide (e.g., GLP-1), pramlintide, or a combination thereof. Drug delivery device 160 may also include a power source, such as a battery, for supplying power to the pump and/or other components of drug delivery device 160. Embodiments are not limited in this context, for example, as drug delivery device 160 may include more or fewer components.

"Analyte sensor" may refer to a sensing and measuring device that may be configured to measure one or more of lactate, ketones, uric acid, sodium, a protein, potassium, alcohol levels, hormone levels, blood glucose, a medication, a drug, a chemotherapy drug, glucagon or a glucagon-like peptide, and/or the like. In some examples, analyte sensor 170 may be configured as a continuous glucose monitor (CGM), which may refer to a wearable device that may at least include a sensor configured to measure blood glucose of a wearer, a transmitter to transmit blood glucose measurement values measured by the sensor (for example, to controller 110 and/or drug delivery device 160), and logic circuitry that controls the sensor and the transmitter. Analyte sensor 170 may be configured, with a processor, a communication device, a memory, a sensing/measuring device, and/or other components (see, for example, FIG. 8). Analyte sensor 170 may output analyte measurement values to any devices that are communicatively coupled to analyte sensor 170 when operating properly (e.g., full power supply, established wireless connectivity, sensing/measuring device operation is proper, and whatever other functions enable the analyte sensor to detect and output an analyte measurement value).

In drug delivery system 105 of FIG. 1, analyte sensor 170 and drug delivery device 160 may be communicatively coupled to one another, and controller 110 and drug delivery device 160 may be communicatively coupled to one another. For example, controller 110 may be wirelessly coupled to drug delivery device 160 via communication link 111 and drug delivery device 160 may be wirelessly coupled to analyte sensor 170 via communication link 112. In some embodiments, controller 110 and analyte sensor 170 may be communicatively coupled to one another, for instance, via communications link 113.

Analyte sensor 170 may provide an analyte measurement value at a specific time during an operational time period to drug delivery device 160. "Analyte measurement value" may refer to a numerical value representative of an amount of an analyte being monitored by an analyte sensor and that has the units in common usage of the analyte being monitored, such as milligrams per deciliter (mg/dL), a percentage, or the like. In an example, analyte sensor 170 may be configured to output an analyte measurement value at a predetermined cadence (e.g., every 1, 2, 5, 6, 8 or 10 minutes) or once within a set period of time (e.g., at time = 0 in a 5 minute period of time). The set period of time in the example of a diabetes treatment plan may be referred to as a "cycle" or "cycle time," which may last 1 minute, or about 1 minute, 5 minutes, or about 5 minutes, about 3 minutes, or about 10 minutes, or the like. A specific time during the operation may be at the beginning of the operational time period.

The outputted analyte measurement value is intended, in this embodiment, for receipt by the drug delivery management application 140 executing on controller 110 and/or automatic analyte control (AAC) application executing on drug delivery device 160 and/or controller 110, but may be shared with other applications, such as an automatic medication delivery application, an artificial pancreas application, and/or the like. The analyte measurement values may include information that includes an analyte measurement value, such a blood glucose measurement value, but also information such as analyte rate of change, status information related to a sensing/measuring device in analyte sensor 170, and/or the like. In some embodiments, drug delivery management application 140 may operate to perform profile adjustment processes according to some embodiments.

Controller 110 may be configured with a processor or processor circuitry 120, a memory unit 130, a user interface 182, a communication device or transceiver 180, and/or the like. Memory unit 130 may store information, such as patient information 132 and/or profile information 134, each of which may include previous analyte measurement values, delivered drug dosages, and/or the like.

In some embodiments, patient information 132 may include information associated with patient 150, including, without limitation, demographic information, physical information (for instance, height, weight, and/or the like), diabetes condition information (for instance, type of diagnosed diabetes (T1D or T2D)), insulin needs (for instance, MDI information, TDI information, insulin types, basal dosage information, bolus dosage information, and/or the like), activity information (for instance, meals and/or meal times, carbohydrate intake, exercise information, and/or the like), insulin sensitivity information, IOB information, response events, BG events (for example, hypoglycemic episodes or hyperglycemic episodes), and/or the like. In some embodiments, at least a portion of patient information 132 may be manually entered by patient 150 or a caregiver, for example, via a user interface of diabetes management application 140. In some embodiments, patient information 132 may include historical information, such as historical values associated with drug dosages, analyte measurement values (for instance, blood glucose measurements of patient 150), mealtimes, carbohydrate intake, exercise times, and/or the like. The historical information may include data from various time ranges, including at least seven days, at least 30 days, at least 60 days, at least the past 90 days, 180 days, 1 year, or more (and any range or value between any of these values, including endpoints).

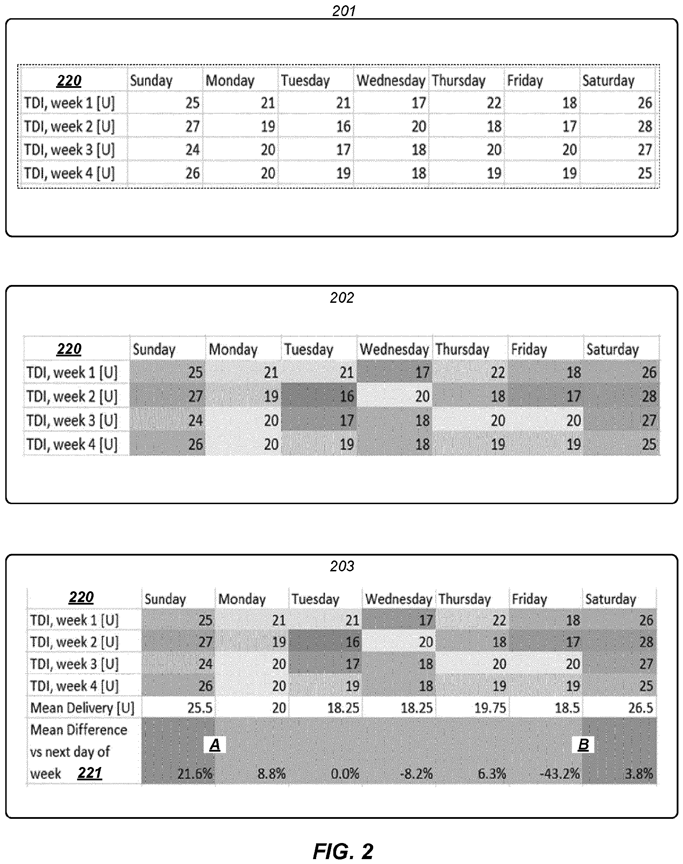

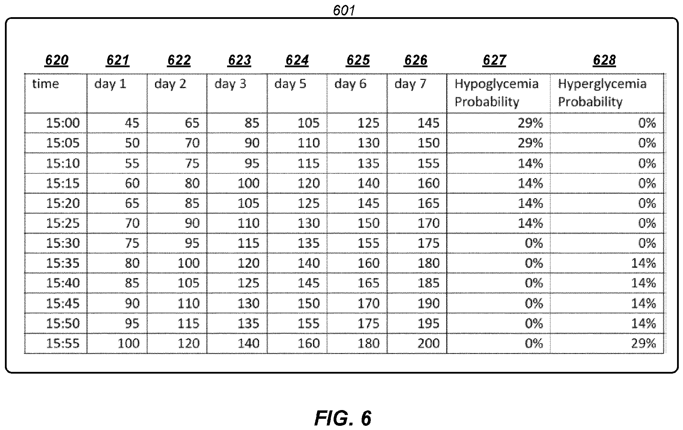

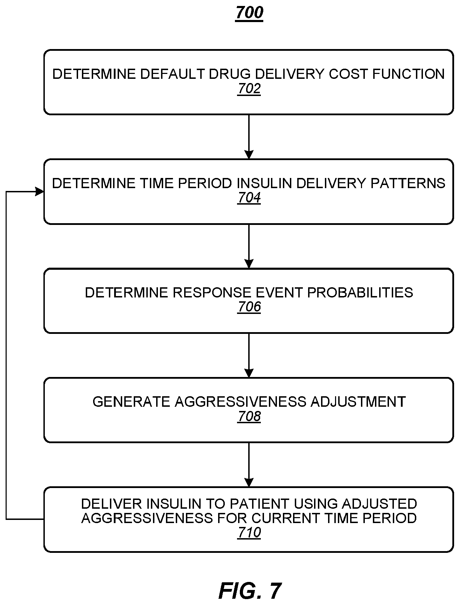

In some embodiments, profile information 134 may include a profile of patient 150 configured according to some embodiments. For example, profile information 134 may be generated using time period dosage information associated with patient (see, for example, FIGS. 2-5) and/or analyte concentration information associated with patient 150 (see, for example, FIGS. 6 and 7). Profile information 134 may be used to determine a profile-adjusted drug dosage and/or a profile-adjusted aggressiveness factor. In some embodiments, profile information 134 may include drug dosage patient information. For example, controller 110 and/or drug delivery management application 140 may receive or access historical patient information showing dosages over certain time periods. Drug delivery management application 140 and/or an AAC (executing on controller 110 and/or drug delivery device 160) may use the profile-adjusted drug dosage to determine and deliver a dosage of the drug.

In various embodiments, profile information 134 may include analyte concentration patient information. In some embodiments, the concentration information may be or may include response events, such as an analyte being over/under a threshold. In one example, controller 110 and/or drug delivery management application 140 may receive or access historical patient information showing analyte concentration(s) over certain time periods. Drug delivery management application 140 and/or an AAC (executing on controller 110 and/or drug delivery device 160) may use the profile-adjusted aggressiveness factor to determine and deliver a dosage of the drug.

Additionally, memory unit 130 may store computer applications, such as a drug delivery management application 140, which may be or may include a medication delivery application (MDA), an automatic analyte control application, an AGC application, an analyte-based drug delivery application, an automatic analyte control application, an artificial pancreas application, an automated insulin delivery (AID) application, an automatic analyte control (AAC), and/or the like, that are executable by processor circuitry 120 and configure processor circuitry 120 to perform different functions according to some embodiments of the present disclosure. Drug delivery management application 140 may be or may include an "automatic analyte control application" that may refer to a computer application that is operable to execute an algorithm operable to use analyte measurement values, including blood glucose measurement values, to determine amounts of a drug to be delivered to a user.

Controller 110 when executing drug delivery management application 140 may be configured to set operational parameters for drug delivery device 160, such as user preferences for drug deliveries (e.g., times, basal and bolus) and notifications/alarms, drug delivery limits, user statistics (e.g., height, weight, gender, age, type of diabetes, or the like) as well as other settings.

Drug delivery device 160 may also be configured with a processor, a memory, a user interface, a communication device, and/or other components (see, for example, FIG. 8). The memory may store information, such as previous analyte measurement values, delivered drug dosages, and the like. Additionally, the memory may store computer applications, such as a medication delivery application (MDA), automatic analyte control application 166, and the like, that are executable by the processor and configure drug delivery device 160 to perform different functions described in the present disclosure. Drug delivery device 160 may during a cycle be configured to use the present analyte measurement value and information related to the present analyte measurement value to determine a dosage of a drug to be delivered during the cycle. The dosage of the drug may be a basal dosage, a bolus dosage (e.g., meal or correction), or a combination of both. "Present analyte measurement value" may refer to the analyte measurement value that is generated by the analyte sensor during a cycle of the drug delivery system. "Information related to the present analyte measurement value" may refer to additional data that is related to the present analyte measurement value such as trend information (i.e., whether the measurement value is increasing, decreasing or level over a period of time), prior analyte measurement values, analyte sensor status (e.g., power level, occlusion status, or the like) and other information.

In some embodiments, a patient may be initialized or "onboarded" to a drug delivery device. For example, an AID application may be operable to, in response to an initial launch (i.e., first time the application is opened on a user device or used by a first-time (i.e., new) patient) or the request to begin the initial dose setting process, the AID application executing on a processor may, for example, set a default initial setting for determining total daily insulin. In one example, the AID application executing on a processor may calculate the user's default initial TDI using a relationship between TDI and weight, such as

$TDI = 0.53 u/kg * weight\ in\ kg,$ where 0.53 is an insulin coefficient that may be based on historical clinical data for a population of newly-diagnosed diabetics, typical clinical guidance by endocrinologists as heuristic rules of thumb that provide reasonable initial glucose control, or the like.

Other methods may be used to determine an initial TDI and/or an adjusted TDI. Non-limiting examples of determining TDI and adjusted TDI during onboarding and subsequent use of an AID device may include embodiments described in U.S. Patent Application Serial No. 16/840,483, titled "Initial Total Daily Insulin Setting for User Onboarding," which is incorporated in the present disclosure as if fully set forth herein.

Although TDI and associated insulin delivery systems and processes are used in certain examples, embodiments are not so limited as this is used for illustrative purposes only. Indeed, dosage standards for other types of drug delivery are contemplated in the present disclosure.

The initial TDI may be adjusted as the patient uses the AID device to maintain blood glucose targets. In some embodiments, profile adjustment processes may use clustering algorithms on long term changes in a patient's drug dosage (for instance, TDI), and utilizing this pattern to improve the input insulin delivery values to an automatic drug delivery process. For example, in one embodiment, profile adjustment processes may operate to use projections of past corrections to analyte concentrations based on current input calibration.

**FIG. 2** illustrates exemplary patient profile information in accordance with the present disclosure. As shown in FIG. 2, patient information 201 may be stored or structured as time period information 220. For example, time period information 220 may be or may include a patient's monthly (or 4-week) insulin delivery patterns with insulin doses (in units "U") for each day of the week. Patient information 202 depicts patient information 201 converted into a relative scale, showing a clear pattern between weekends (Sunday/Saturday) versus weekdays.

In one embodiment, the clustering of dosage patterns may be illustrated in patient information 203 by assessing the average daily TDI for every 7th day, and assessing whether mean TDI is more than X% (such as 15%) greater than the TDI of the previous day, or mean TDI of the previous day based on historical data. In patient information 215, two clear transitions at points A and B are shown with deviations greater than the example threshold of 15%, with variations between the transitions being comparatively minimal. Transition A is between Sunday and Monday (i.e., transitioning from the weekend to weekdays) and transition B is between Friday and Saturday (i.e., transitioning from weekdays to the weekend). One or more transitions may be present in a particular patient's data, but in this exemplary patient data, two transitions were identified using an exemplary threshold of 15% variation.

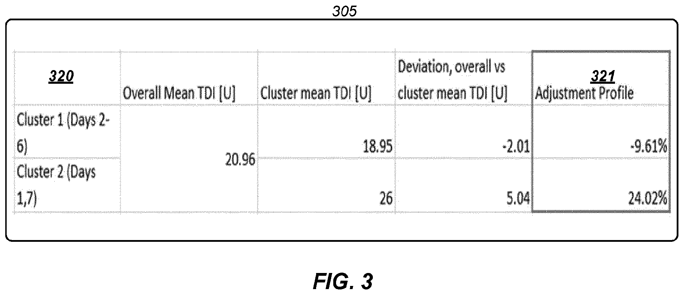

**FIG. 3** illustrates patient adjustment profile information in accordance with the exemplary patient profile information shown in Fig. 2. In some embodiments, the four 7-day cycles (28 days of history) depicted in patient information 201-203 may be used to determine patient profile 305 of FIG. 3 that includes clusters 320 of Cluster 1 (days 2-6 (i.e., weekdays)) and Cluster 2 (days 1 and 7 (i.e., weekend days)). An adjustment profile 321 to overall input TDIs may be determined, which can vary based on characteristics of the identified clusters 320 in the patient profile information.

This adjustment profile 321 with a period of 7 days can be applied to the average input TDI of an AID process based on overall insulin delivery history to improve the AID process, for instance, by providing a capability to compensate for long term patterns in the patient's insulin needs. The use of an adjustment profile may result in a modification of the TDI based basal that is utilized by the AID process in days 2-6 (i.e., weekdays in this example) as compared with days 1 and 7 (i.e., weekends in this example). For example, for adjustment profile 321, the AID process may receive approximately 26/20.96=1.24, or 24% increased TDI based basal during Cluster 2 (i.e., the weekend) and approximately 18.95/20.96=0.904, or ~10% decreased TDI based basal during Cluster 1 (i.e., weekdays). Profile adjustment changes in TDI may also impact the AID process estimates of various other TDI-dependent functions, such as an insulin-on-board (IOB) constraint, cost functions, and/or the like, resulting in an overall increased or decreased insulin delivery compared to the mean.

In some embodiments, the overall mean TDI may be calculated by the following Equation (1):

$$TDI_{overall} = \frac{\sum_{i=k-m}^{k} TDI(i)}{m+1},$$

where k = the current day, and m is a tunable parameter that can be set to 27, as in the example of FIG. 3, but can have a different range, such as, for example, anywhere between 7-55, in 7 day increments.

In various embodiments, the cluster mean TDI may be calculated according to the following Equation (2):

$$TDI_C = \frac{\sum_{i=p-n}^{p} TDI_C(i)}{n+1},$$

where p = the duration (for instance, in days) of cluster number C (identified in 7 day increments for example), and C = the cluster number. The identification of the cluster may be calculated according to the following Equation (3):

$$C(i) = \begin{cases} C(i-1) + 1 & \frac{TDI(i) - TDI(i-1)}{TDI(i-1)} > th \\ C(i-1) & \frac{TDI(i) - TDI(i-1)}{TDI(i-1)} \leq th \end{cases},$$

where *th* is a tunable threshold or cluster criterion, which, for example, may range from 10% to 100%.

The adjustment profile for each cluster may be calculated according to the following Equation (4):

$$b_{adj}(r) = \frac{TDI_{C\_r} - TDI_{C_{r-1}}}{TDI_{C_{r-1}}},$$

where r is the $r^{th}$ unique cluster value.

Although days, weeks, months, weekdays, and weekend days are used in the examples depicted in association with FIGS. 2 and 3, embodiments are not so limited. For example, profile adjustments may be based on any time period, periodicity, and/or the like. Non-limiting examples of time periods may include seasons, holidays, life events (birthdays, anniversaries, vacations, and/or the like), and/or combinations thereof. For example, a time period may include a yearly time period with clusters based on seasons (i.e., macro-adjustment pattern) as well as clusters based on days of the week (i.e., micro-adjustment pattern).

In various embodiments, the adjustment factor determination may be dynamically updated. For example, a profile adjustment process may repeatedly do a 28-day (or other time period duration) history analysis, with the patterns being updated every 7 days after the initial 28 days. The profile adjustment process may then dynamically change adjustment profile based on 7 days of new data to update the existing adjustment profile, clusters, and/or the like. For example, a patient with an existing adjustment profile may engage in a new exercise activity on Tuesdays and Thursdays and the profile adjustment process may eventually update the adjustment profile for this new activity (and, therefore, TDI) pattern.

Included herein are one or more logic flows representative of exemplary methodologies for performing novel aspects of the disclosed architecture. While, for purposes of simplicity of explanation, the one or more methodologies shown herein are shown and described as a series of acts, those skilled in the art will understand and appreciate that the methodologies are not limited by the order of acts. Some acts may, in accordance therewith, occur in a different order and/or concurrently with other acts from that shown and described herein. For example, those skilled in the art will understand and appreciate that a methodology could alternatively be represented as a series of interrelated states or events, such as in a state diagram. Moreover, not all acts illustrated in a methodology may be required for a novel implementation.

A logic flow may be implemented in software, firmware, hardware, or any combination thereof. In software and firmware embodiments, a logic flow may be implemented by computer executable instructions stored on a non-transitory computer readable medium or machine readable medium. The embodiments are not limited in this context.

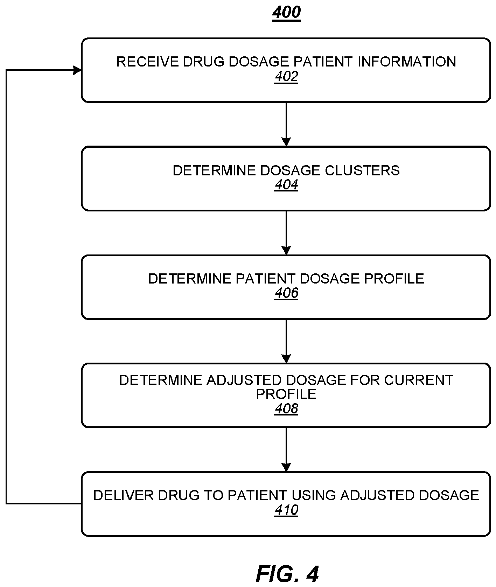

FIG. 4 illustrates an embodiment of a logic flow 400. Logic flow 400 may be representative of some or all of the operations executed by one or more embodiments described herein, such as devices of operating environment 100 and/or 800. For example, logic flow 400 may be a profile adjustment process performed by controller 110 of FIG. 1.

At block 402, logic flow 400 may receive drug dosage patient information. For example, controller 110 may receive or access historical patient information showing dosages over certain time periods. In some embodiments, a time period may be or may include an hour, a day, parts of a day (for instance, morning, noon, night, commute, lunch, and/or the like), a week, weekdays, weekends, a month, a year, holidays, personal events, seasons, and/or the like. In some embodiments, a user may specify (for instance, via user interface 182) a time period and/or a duration (for instance, use one week, one month, one year of historical information). In some embodiments, controller 110 may use a default cyclical time period (for instance, one week) and duration (for instance, one month).

Logic flow 400 may determine dosage clusters at block 404. For example, cluster criterion may be specified for segmenting a time period into clusters. In one example, a cluster criterion may be a percentage (or other measure) difference between doses delivered between parts of a time period. For example, for a week, a profile adjustment process may determine a percentage difference in a daily dose of a drug for days of the week. If a day of the week has a dosage difference or deviation over the threshold (for instance, 15%) when compared with other days of the week (for example, consecutive or immediately following days of the week), then a cluster may be determined. In some embodiments, the dosage difference may be based on a comparison of the same day (e.g., comparing the average of a Wednesday to the average of another Wednesday, comparing the average of a Saturday to the average of another Saturday, and/or the like) or category of day (e.g., comparing the average of a weekday to the average of another weekday, comparing the average of a weekend day to the average of another weekend day, comparing the average of a holiday to the average of another holiday, and/or the like). A deviation threshold may have various values, such as a 1% deviation, a 5% deviation, 10% deviation, 15% deviation, 20% deviation, 30% deviation, 40% deviation, 50% deviation, 75% deviation, 90% deviation, 100% deviation, 500% deviation, and any value or range between any two of these values (including endpoints). In some embodiments, the deviation threshold may be based on other measures, such as dosage units. A time period may be segmented into any number of clusters depending on the cluster criterion. For example, a week may be segmented into seven clusters, one for each day of the week.

At block 406, logic flow 400 may determine a patient adjustment profile. For example, a profile adjustment process may operate to determine an adjustment profile to adjust a base or default dosage amount. In some embodiments, an overall dosage mean may be determined for the patient and a cluster dosage mean may be determined for each cluster. The adjustment profile may be a ratio or other relationship between the overall dosage mean and the cluster mean. For example, an overall dosage mean for a drug may be 20 units, a first cluster dosage mean for a first cluster may be 25 units, and a second cluster dosage mean for a second cluster may be 15 units. An adjustment profile for the first cluster may equal 125% = (25/20)*100 and an adjustment profile for the second cluster may equal 75% = 15/20. Accordingly, future dosages for the first cluster may be set to (original value) * 1.25 (i.e., 25% greater than earlier time periods for this particular cluster) and future dosages for the second cluster may be set to (original value) * .75 (i.e., 25% less than earlier time periods for this particular cluster). In some embodiments, the original value may be a basal dosage, default dosage, and/or the like. In various embodiments, the default dosage may be a basal dosage for the patient.

Although percentage reductions/additions are used in some examples, embodiments are not so limited. For example, an adjustment profile may be used to directly add or subtract form a default or basal dosage for a cluster.

At block 408, logic flow 400 may determine an adjusted dosage for a current profile. For example, controller 110 may determine the current cluster for a current dosing cycle and may calculate the appropriate dosage for the cluster based on the adjustment profile (for instance, adjusted dosage = (original value) * 0.75 for the second cluster example above).

In some embodiments, controller 110 may provide a message or alert to patient that an adjusted dosage is being used. In various embodiments, controller 110 may provide cluster information to a user, such as determined clusters and associated adjustment profiles. In exemplary embodiments, controller 110 may require that a user (or healthcare professional) accept the cluster-based adjustments to the drug dosages, for example by clicking a confirmation button on a user interface of controller 110.

At block 410, logic flow 400 may deliver the drug to the patient using the adjusted dosage for the current cluster.

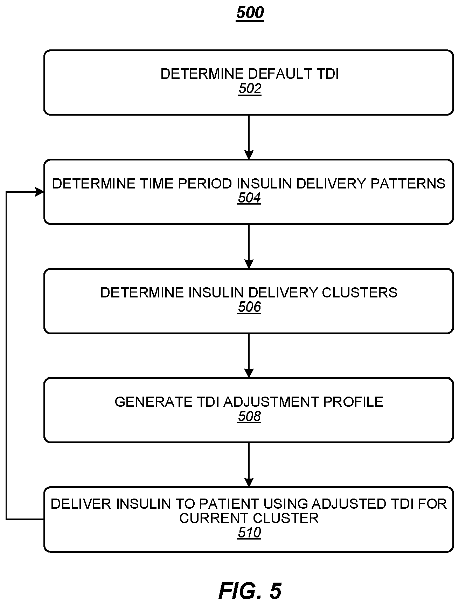

FIG. 5 illustrates an embodiment of a logic flow 500. Logic flow 500 may be representative of some or all of the operations executed by one or more embodiments described herein, such as devices of operating environment 100 and/or 800. For example, logic flow 500 may be a profile adjustment process performed by controller 110 of FIG. 1 for an AID drug delivery device 160.

At block 502, logic flow 500 may determine a default TDI. For example, controller 110 may determine a default or basal TDI (i.e., an "original value") for a user, such as through an on-boarding process. The default TDI may include adjustments for certain factors, such as age, activity level, and/or learned blood glucose control needs.

Logic flow 500 may determine time period insulin delivery patterns at block 504. For example, as shown in FIG. 2, patient information 132 may include insulin delivery patterns of a patient based on one or more time periods, such as a monthly insulin delivery pattern of patient information 201. At block 506, logic flow 500 may determine insulin delivery clusters. For example, a cluster criterion may be determined for segmenting a time period into clusters. In the example of FIG. 2, a cluster criterion may include whether the TDI for a day is more than X% (such as 15%) greater than the TDI for a previous day or days. In some embodiments, the TDI may include a mean value over a specific time period, such as multiple days, multiple weeks, a month, and/or the like. Various other cluster criterion may be or may include total dosage minimum, total dosage maximum, dosage deviation from a mean, activity levels, location, combinations thereof, and/or the like. For example, as depicted in FIG. 3, clusters 320 for weekdays and weekend days were determined for the patient.

At block 508, logic flow 500 may determine a TDI adjustment profile. For example, controller may determine an adjustment profile for each cluster, such as a percentage of TDI or basal insulin dose to administer over a cluster time period. For example, for Cluster 1 and Cluster 2 of FIG. 3, about 90% of TDI may be administered on weekdays (Cluster 1) and about 124% of TDI may be administered on weekend days (Cluster 2), as compared with prior weekday deliveries and prior weekend deliveries, respectively.

For instance, adjustment profile 321 of FIG 3, with a time period of 7 days, can be applied to the average input TDI of an AID process based on overall insulin delivery history to improve the AID process, for instance, by providing a capability to compensate for long term patterns in the patient's insulin needs. The use of an adjustment profile may result in a modification of the TDI based basal that is utilized by the AID process in days 2-6 (i.e., weekdays) as compared with days 1 and 7 (i.e., weekends). For example, for adjustment profile 321, the AID process may receive approximately 26/20.96=1.24, or 24% increased TDI based basal during Cluster 2 (i.e., the weekend) and approximately 18.95/20.96=0.904 (or ~10%) decreased TDI based basal during Cluster 1 (i.e., weekdays). Profile adjustment changes in TDI may also impact the AID process estimates of various other TDI-dependent functions, such as an insulin-on-board (IOB) constraint, cost functions, and/or the like, resulting in an overall increased or decreased insulin delivery compared to the mean.

In some embodiments, the overall mean TDI may be calculated by Equation (1). In various embodiments, the cluster mean TDI may be calculated according to Equation (2). The identification of the cluster may be calculated according to the following Equation (3), where *th* is a tunable threshold or cluster criterion, which, for example, may range from 10% to 100%. In exemplary embodiments, the adjustment profile for each cluster may be calculated according to Equation (4).

At block 510, logic flow 500 may deliver insulin to the patient using the adjusted TDI for the current cluster. For example, controller 110 may determine the current cluster (for instance, a weekend day) and determine the adjustment profile for the cluster. The adjusted TDI may be determined by applying the adjustment profile to the default TDI for the day (for instance, 26 units for Sunday) to derive the adjusted TDI, for example, (26 units) * (1.24) = 32.24 units. As explained earlier, modified TDI may be applied to both basal and bolus deliveries or may be applied to only one of basal or bolus. For example, the additional insulin delivered after applying the adjustment profile may be delivered via basal only, by way of example.

In some embodiments, a drug delivery system may perform a profile-based aggressiveness adjustment process to determine a dosage of a drug for delivery to a patient based on one or more patient characteristics. In some embodiments, the one or more patient characteristics may include a patient analyte concentration (i.e., a concentration of the analyte in the blood of the patient), a patient drug concentration, a response event, and/or the like. For example, for a blood glucose control system, the profile-based aggressiveness adjustment process may operate to adjust the system's aggressiveness in real time based on a history of the user's previous glucose concentrations.

In some embodiments, drug delivery system (for instance, via a controller, drug delivery device, an AAC application, and AID application, a drug delivery management application, and/or the like) may operate to determine response events. For example, an AID system may operate to determine hypoglycemia, hyperglycemia, and within range (e.g., within a target blood glucose value range) response events.

FIG. 6 illustrates patient profile information in accordance with the present disclosure. More specifically, FIG. 6 illustrates patient information 601 in the form of a table or matrix of a patient's percentage time in hypoglycemia and hyperglycemia. In FIG. 6, the patient information 601 is determined at time period (i.e., every 5-minute cycle) within a time range of 3:00 pm (15:00) to 4:00 pm (16:00) 620 over a duration of 7 days (i.e., day 1 - day 7) 621-626. Although a specific time period, time range, and duration are depicted in FIG. 6, embodiments are not so limited, as patient profile information 601 may be captured at various time periods (time-based, cycle-based, event-based, and/or the like), for various time (or event) ranges, for different durations (days, weeks, months, years, specified time periods (e.g., seasons, quarters, and/or the like), and/or the like), and/or different clusters. Embodiments are not limited in this context.

In some embodiments, matrix 601 may be generated by an AID system configured according to the present disclosure. For example, in various embodiments, response event probabilities for a hypoglycemia probability 627 and/or hyperglycemia probability 628 may be determined. In general, hypoglycemia probability 627 and/or hyperglycemia probability 628 may be a probability or risk of a patient entering a hypoglycemia or hyperglycemia, respectively, state based on the profile information. Hypoglycemia probability 627 and/or hyperglycemia probability 628 may, in various embodiments, be calculated based on a duration or a portion of a time period (e.g., the number of days in the previous week) that the user had experienced glucose concentrations in hypoglycemic or hyperglycemic range within the current control cycle. For instance, if, in the past 7 days, the user experienced glucose concentrations below 70 mg/dL for 3 days, the probability of hypoglycemia for this user would be approximately 42.9%.

In various embodiments, the profile-based aggressiveness adjustment process may modify, update, or otherwise change the aggressiveness factor of drug delivery based on the probabilities 627, 628 of matrix 601. In one non-limiting example of an AID system, such as an Omnipod® system, the aggressiveness factor may be an update to the Q:R ratio (e.g., weighted coefficients of a cost factor) used to determine insulin delivery dosages. In general, the Q:R ratio may be or may include relative weights of a glucose excursion penalty and an insulin excursion penalty.

In various embodiments, an estimated aggressiveness factor R'(i) may be determined for a current cycle i according to the following Equation (5):

$$R'(i) = 9000 \cdot \left(1 + 0.2 \cdot P_{hypo}(i)\right)^2 \cdot \left(1 - 0.2 \cdot P_{hyper}(i)\right)^2,$$

where $P_{hypo}$ is the probability of a hypoglycemic response event 627 and $P_{hyper}$ is the probability of a hyperglycemic response event 628. In the example embodiments of Equation (5), the maximum adjustment is limited to no more than +20% (i.e., +0.2) or -20% (i.e., -0.2) of the original setting, applied by a factor of 2. In some embodiments, the constant factors (i.e., 9000, 0.2, factor of 2 (e.g., squared factor) may be adjusted and/or tuned for a particular device, patient, and/or the like. In one example, the '9000' constant factor may be a value of a default QR ratio that may be adjusted for a particular user. In various embodiments, there may be an adjustment threshold for a probability of a response event (such as hypoglycemia and/or hyperglycemia) required to trigger an aggressiveness adjustment (e.g., probability must be over 5%, 10%, 20%, 50%, 75%, and/or the like).

The actual R utilized by the algorithm in the current cycle, R(i) can be calculated as a function of the previous R utilized in the algorithm and the estimated R' in the current cycle according to the following Equation (6):

$$R(i) = f(R(i-1), R(i)').$$

In one example embodiment, Equation (6) may be a weighted average, as shown in the following Equation (7):

$$f(R(i-1), R(i)') = 0.8 \cdot R(i-1) + 0.2 \cdot R(i)'.$$

The functions of Equations (6) and (7) may be associated with various forms and/or filters, including, without limitation, a Kalman filter.

Although Equations (5)-(7) depict the aggressiveness factor R being determined every cycle (i), embodiments are not so limited as the aggressiveness factor may be determined at different intervals, events, and/or the like. For example, the aggressiveness factor may be recalculated per "blocks" of time, such as every 5 minutes, every 30 minutes, every hour, every 5 hours, or longer. Longer calculation blocks may allow for larger datasets to be utilized for calculation of hyperglycemia and hypoglycemia probabilities, and reduce the impact of any one point of hyper or hypoglycemic risk. In various embodiments, the blood glucose history may be applied as a fading history over time, where the glucose values in older dates are weighted less for the calculation of hyperglycemia and hypoglycemia probabilities than the latest datasets. For example, a weight factor may be applied to probabilities used in Equation (5), such as multiplying $P_{hypo}$ and/or $P_{hyper}$ by a factor (e.g., 0.2, 0.5, 0.9, and/or the like) based on how distant they are from a current cycle, block, and/or the like. In various embodiments, hypoglycemic or hyperglycemic incidents earlier in the relevant history range may be weighted less heavily whereas the more recent incidents may be weighted more heavily. For instance, if the user experienced a hypoglycemic glucose concentration in day 1 of the previous 7 days, this may actually be incorporated as 0.8 incidents in the assessment of probability. Conversely, a hypoglycemic incident in day 7 of the previous 7 days may be incorporated as 1.2 incidents in the assessment of probability.

In some embodiments, the aggressiveness factor R may be weighted within a cost equation used by a drug delivery system to determine a drug delivery dosage, for example, to indirectly alter R within the cost equation. For instance, a new or more recent R may be weighted at 20% and a previous R may be weighted by 80%. In general, the more hypoglycemic probability, decrease the aggressiveness, the more hyperglycemic probability, increase aggressiveness.

In some embodiments, the response event probabilities may be used in a cost equation to directly affect a drug delivery rate (for instance, instead of adjusting the aggressiveness factor). For instance, for an AID system, the AID algorithm's basal rate may be adjusted up/down based on past probabilities of hypoglycemic events and/or hyperglycemic events. In other embodiments, response event probabilities may be used to determine coefficients or other values for modifying drug delivery algorithm dosage determinations. Embodiments are not limited in this context.

**FIG. 7** illustrates an embodiment of a logic flow 700. Logic flow 700 may be representative of some or all of the operations executed by one or more embodiments described herein, such as devices of operating environment 100 and/or 800. For example, logic flow 700 may be a profile aggressiveness adjustment process performed by controller 110 of FIG. 1 for an AID drug delivery device 160.

At block 702, logic flow 700 may determine a default drug delivery cost function. For example, controller 110 may determine a default or basal TDI (i.e., an "original value") for a user, such as through an on-boarding process. The default TDI may include adjustments for certain factors, such as age, activity level, and/or learned blood glucose control needs. In some embodiments, the default drug delivery cost function may include an aggressiveness factor to determine the speed of response of an AID algorithm to changes in glucose concentration.

Logic flow 700 may determine time period insulin delivery patterns at block 704. For example, as shown in FIGS. 3 and 6, patient information may be determined that is associated with drug delivery values during time clusters and/or blocks (FIG. 3) and/or patient blood glucose levels (FIG. 6). For instance, a patient may have a different response to insulin (e.g., more likely to have a lower response) during a certain time period of a day (for instance, 12:00 pm) versus another time period (for instance, 8:00 am). At block 706, logic flow 700 may determine response event probabilities. For example, response event probabilities may be determined for specific events, such as hypoglycemic probabilities 627 and hyperglycemic probabilities 628, for certain time periods or clusters over a duration (for instance, 7 days).

At block 708, logic flow 700 may determine an aggressiveness adjustment. For example, Equations (5) and (6) may be used to determine an aggressiveness factor R to be used in a cost function to determine an insulin delivery dosage at a particular delivery cycle. At block 710, logic flow 700 may deliver insulin to the patient using the adjusted aggressiveness factor for the current cycle. For example, a drug delivery device may deliver insulin to a patient using a cost function having an aggressiveness factor coefficient determined based on response probabilities according to some embodiments.

**FIG. 8** illustrates a functional block diagram of a system example suitable for implementing the example processes and techniques described herein.

The operating environment 800 may be or may include an automatic drug delivery system that may include components such as an automatic drug delivery system that is configured to determine a drug dosage and deliver the dosage of the drug without any user interaction, or in some examples, limited user interaction, such as in response to a user depressing a button to indicate measurement of blood glucose or another analyte, or the like. "Drug delivery system environment" may refer to a computing and sensing environment that includes cloud based services, a drug delivery system (that may include a controller, a drug delivery device, and an analyte sensor) and optionally additional devices. The components of the drug delivery system environment may cooperate to provide present analyte measurement values or at least accurate estimates of present analyte measurement values to facilitate calculation of optimal drug dosages for a user.

The automatic drug delivery system 800 may implement (and/or provide functionality for) a medication delivery algorithm, such as an artificial pancreas (AP) application, to govern or control automated delivery of a drug or medication, such as insulin, to a user (e.g., to maintain euglycemia - a normal level of glucose in the blood). The drug delivery system 800 may be an automated drug delivery system that may include a wearable automatic drug delivery device 802, an analyte sensor 803, and a management device (for instance, a PDM, smart phone, table computing device, and/or the like) 805.

The system 800, in an optional example, may also include a smart accessory device 807, such as a smartwatch, a personal assistant device or the like, which may communicate with the other components of system 800 via either a wired or wireless communication links 891-893.

The management device 805 may be a computing device such as a smart phone, a tablet, a personal diabetes management device, a dedicated diabetes therapy management device, or the like. In an example, the management device (PDM) 805 may include a processor 851, a management device memory 853, a user interface 858, and a communication device 854. The management device 805 may contain analog and/or digital circuitry that may be implemented as a processor 851 for executing processes based on programming code stored in the management device memory 853, such as the medication delivery algorithm or application (MDA) 859, to manage a user's blood glucose levels and for controlling the delivery of the drug, medication, or therapeutic agent to the user as well as other functions, such as calculating carbohydrate-compensation dosage, a correction bolus dosage and the like as discussed above. The management device 805 may be used to program, adjust settings, and/or control operation of the wearable automatic drug delivery device 802 and/or the analyte sensor 803 as well as the optional smart accessory device 807.

The processor 851 may also be configured to execute programming code stored in the management device memory 853, such as the MDA 859. The MDA 859 may be a computer application that is operable to deliver a drug based on information received from the analyte sensor 803, the cloud-based services 811 and/or the management device 805 or optional smart accessory device 807. The memory 853 may also store programming code to, for example, operate the user interface 858 (e.g., a touchscreen device, a camera or the like), the communication device 854 and the like. The processor 851 when executing the MDA 859 may be configured to implement indications and notifications related to meal ingestion, blood glucose measurements, and the like. The user interface 858 may be under the control of the processor 851 and be configured to present a graphical user interface that enables the input of a meal announcement, adjust setting selections and the like as described above.

In a specific example, when the MDA 859 is an artificial pancreas (AP) application, the processor 851 is also configured to execute a diabetes treatment plan (which may be stored in a memory) that is managed by the MDA 859 stored in memory 853. In addition to the functions mentioned above, when the MDA 859 is an AP application, it may further provide functionality to enable the processor 851 to determine a carbohydrate-compensation dosage, a correction bolus dosage and determine a basal dosage according to a diabetes treatment plan. In addition, as an AP application, the MDA 859 provides functionality to enable the processor 851 to output signals to the wearable automatic drug delivery device 802 to deliver the determined bolus and basal dosages described with reference to the examples of FIGS. 1-4.

The communication device 854 may include one or more transceivers such as Transceiver A 852 and Transceiver B 856 and receivers or transmitters that operate according to one or more radio-frequency protocols. In the example, the transceivers 852 and 856 may be a cellular transceiver and a Bluetooth® transceiver, respectively. For example, the communication device 854 may include a transceiver 852 or 856 configured to receive and transmit signals containing information usable by the MDA 859.

The wearable automatic drug delivery device 802, in the example system 800, may include a user interface 827, a controller 821, a drive mechanism 825, a communication device 826, a memory 823, a power source/energy harvesting circuit 828, device sensors 884, and a reservoir 824. The wearable automatic drug delivery device 802 may be configured to perform and execute the processes described in the examples of FIGS. 4, 5, and 7 without input from the management device 805 or the optional smart accessory device 807. As explained in more detail, the controller 821 may be operable, for example, implement the processes of FIGS. 4, 5, and 7 as well as determine an amount of insulin delivered, IOB, insulin remaining, and the like. The controller 821 alone may implement the processes of FIGS. 4, 5, and 7 as well as determine an amount of insulin delivered, IOB, insulin remaining, and the like, such as control insulin delivery, based on an input from the analyte sensor 804.

The memory 823 may store programming code executable by the controller 821. The programming code, for example, may enable the controller 821 to control expelling insulin from the reservoir 824 and control the administering of doses of medication based on signals from the MDA 829 or, external devices, if the MDA 829 is configured to implement the external control signals.

The reservoir 824 may be configured to store drugs, medications or therapeutic agents suitable for automated delivery, such as insulin, morphine, blood pressure medicines, chemotherapy drugs, or the like.

The device sensors 884 may include one or more of a pressure sensor, a power sensor, or the like that are communicatively coupled to the controller 821 and provide various signals. For example, the pressure sensor may be coupled to or integral with a needle/cannula insertion component (which may be part of the drive mechanism 825) or the like. In an example, the controller 821 or a processor, such as 851, may be operable to determine that a rate of drug infusion based on the indication of the fluid pressure. The rate of drug infusion may be compared to an infusion rate threshold, and the comparison result may be usable in determining an amount of insulin onboard (IOB) or a total daily insulin (TDI) amount.

In an example, the wearable automatic drug delivery device 802 includes a communication device 826, which may be a receiver, a transmitter, or a transceiver that operates according to one or more radio-frequency protocols, such as Bluetooth, Wi-Fi, a near-field communication standard, a cellular standard, or the like. The controller 821 may, for example, communicate with a personal diabetes management device 805 and an analyte sensor 803 via the communication device 826.

The wearable automatic drug delivery device 802 may be attached to the body of a user, such as a patient or diabetic, at an attachment location and may deliver any therapeutic agent, including any drug or medicine, such as insulin or the like, to a user at or around the attachment location. A surface of the wearable automatic drug delivery device 802 may include an adhesive to facilitate attachment to the skin of a user as described in earlier examples.

The wearable automatic drug delivery device 802 may, for example, include at least one housing, a reservoir 824 for storing the drug (such as insulin), a needle or cannula (not shown in this example) for delivering the drug into the body of the user (which may be done subcutaneously, intraperitoneally, or intravenously), and a drive mechanism 825 for transferring the drug from the reservoir 824 through a needle or cannula and into the user. The drive mechanism 825 may be fluidly coupled to reservoir 824, and communicatively coupled to the controller 821.

The wearable automatic drug delivery device 802 may further include a power source 828, such as a battery, a piezoelectric device, other forms of energy harvesting devices, or the like, for supplying electrical power to the drive mechanism 825 and/or other components (such as the controller 821, memory 823, and the communication device 826) of the wearable automatic drug delivery device 802.

In some examples, the wearable automatic drug delivery device 802 and/or the management device 805 may include a user interface 858, respectively, such as a keypad, a touchscreen display, levers, light-emitting diodes, buttons on a housing of the management device 805, a microphone, a camera, a speaker, a display, or the like, that is configured to allow a user to enter information and allow the management device 805 to output information for presentation to the user (e.g., alarm signals or the like). The user interface 858 may provide inputs, such as a voice input, a gesture (e.g., hand or facial) input to a camera, swipes to a touchscreen, or the like, to processor 851 which the programming code interprets.

When configured to communicate to an external device, such as the PDM 805 or the analyte sensor 804, the wearable automatic drug delivery device 802 may receive signals over the wired or wireless link 894 from the management device (PDM) 805 or 808 from the analyte sensor 804. The controller 821 of the wearable automatic drug delivery device 802 may receive and process the signals from the respective external devices as described with reference to the examples of FIGS. 4, 5, and 7 as well as implementing delivery of a drug to the user according to a diabetes treatment plan or other drug delivery regimen.

In an operational example, the processor 821 when executing the MDA 859 may output a control signal operable to actuate the drive mechanism 825 to deliver a carbohydrate-compensation dosage of insulin, a correction bolus, a revised basal dosage or the like as described with reference to the examples of FIGS. 4, 5, and 7.

The smart accessory device 807 may be, for example, an Apple Watch®, other wearable smart device, including eyeglasses, provided by other manufacturers, a global positioning system-enabled wearable, a wearable fitness device, smart clothing, or the like. Similar to the management device 805, the smart accessory device 807 may also be configured to perform various functions including controlling the wearable automatic drug delivery device 802. For example, the smart accessory device 807 may include a communication device 874, a processor 871, a user interface 878 and a memory 873. The user interface 878 may be a graphical user interface presented on a touchscreen display of the smart accessory device 807. The memory 873 may store programming code to operate different functions of the smart accessory device 807 as well as an instance of the MDA 879. The processor 871 that may execute programming code, such as site MDA 879 for controlling the wearable automatic drug delivery device 802 to implement the FIGS. 4, 5, and 7 examples described herein.

The analyte sensor 803 may include a controller 831, a memory 832, a sensing/measuring device 833, a user interface 837, a power source/energy harvesting circuitry 834, and a communication device 835. The analyte sensor 803 may be communicatively coupled to the processor 851 of the management device 805 or controller 821 of the wearable automatic drug delivery device 802. The memory 832 may be configured to store information and programming code, such as an instance of the MDA 836.

The analyte sensor 803 may be configured to detect multiple different analytes, such as lactate, ketones, uric acid, sodium, potassium, alcohol levels or the like, and output results of the detections, such as measurement values or the like. The analyte sensor 803 may, in an example, be configured to measure a blood glucose value at a predetermined time interval, such as every 30 seconds, every 1 minute, every 5 minutes, every 10 minutes, every 30 minutes, every hour, every cycle, and/or other time intervals. The communication device 835 of analyte sensor 803 may have circuitry that operates as a transceiver for communicating the measured blood glucose values to the management device 805 over a wireless link 895 or with wearable automatic drug delivery device 802 over the wireless communication link 808. While called an analyte sensor 803, the sensing/measuring device 833 of the analyte sensor 803 may include one or more additional sensing elements, such as a glucose measurement element a heart rate monitor, a pressure sensor, or the like. The controller 831 may include discrete, specialized logic and/or components, an application-specific integrated circuit, a microcontroller or processor that executes software instructions, firmware, programming instructions stored in memory (such as memory 832), or any combination thereof.

Similar to the controller 821, the controller 831 of the analyte sensor 803 may be operable to perform many functions. For example, the controller 831 may be configured by the programming code stored in the memory 832 to manage the collection and analysis of data detected the sensing and measuring device 833.

Although the analyte sensor 803 is depicted in FIG. 8 as separate from the wearable automatic drug delivery device 802, in various examples, the analyte sensor 803 and wearable automatic drug delivery device 802 may be incorporated into the same unit. That is, in various examples, the sensor 803 may be a part of the wearable automatic drug delivery device 802 and contained within the same housing of the wearable automatic drug delivery device 802 (e.g., the sensor 803 or, only the sensing/measuring device 833 and memory storing related programming code may be positioned within or integrated into, or into one or more components, such as the memory 823, of, the wearable automatic drug delivery device 802). In such an example configuration, the controller 821 may be able to implement the process examples of FIGS. 4, 5, and 7 alone without any external inputs from the management device 805, the cloud-based services 811, another sensor (not shown), the optional smart accessory device 807, or the like.

The communication link 815 that couples the cloud-based services 811 to the respective devices 802, 803, 805 or 807 of system 800 may be a cellular link, a Wi-Fi link, a Bluetooth link, or a combination thereof. Services provided by cloud-based services 811 may include data storage that stores anonymized data, such as blood glucose measurement values, historical IOB or TDI, prior carbohydrate-compensation dosage, and other forms of data. In addition, the cloud-based services 811 may process the anonymized data from multiple users to provide generalized information related to TDI, insulin sensitivity, IOB and the like.

The wireless communication links 808, 891, 892, 893, 894 and 895 may be any type of wireless link operating using known wireless communication standards or proprietary standards. As an example, the wireless communication links 808, 891, 892, 893, 894 and 895 may provide communication links based on Bluetooth®, Zigbee®, Wi-Fi, a near-field communication standard, a cellular standard, or any other wireless protocol via the respective communication devices 854, 874, 826 and 835.

Software related implementations of the techniques described herein, such as the processes examples described with reference to FIGS. 4, 5, and 7 may include, but are not limited to, firmware, application specific software, or any other type of computer readable instructions that may be executed by one or more processors. The computer readable instructions may be provided via non-transitory computer-readable media. Hardware related implementations of the techniques described herein may include, but are not limited to, integrated circuits (ICs), application specific ICs (ASICs), field programmable arrays (FPGAs), and/or programmable logic devices (PLDs). In some examples, the techniques described herein, and/or any system or constituent component described herein may be implemented with a processor executing computer readable instructions stored on one or more memory components.

In addition, or alternatively, while the examples may have been described with reference to a closed loop algorithmic implementation, variations of the disclosed examples may be implemented to enable open loop use. The open loop implementations allow for use of different modalities of delivery of insulin such as smart pen, syringe or the like. For example, the disclosed AP application and algorithms may be operable to perform various functions related to open loop operations, such as the generation of prompts requesting the input of information such as weight or age. Similarly, a dosage amount of insulin may be received by the AP application or algorithm from a user via a user interface. Other open-loop actions may also be implemented by adjusting user settings or the like in an AP application or algorithm.

Some examples of the disclosed device or processes may be implemented, for example, using a storage medium, a computer-readable medium, or an article of manufacture which may store an instruction or a set of instructions that, if executed by a machine (i.e., processor or controller), may cause the machine to perform a method and/or operation in accordance with examples of the disclosure. Such a machine may include, for example, any suitable processing platform, computing platform, computing device, processing device, computing system, processing system, computer, processor, or the like, and may be implemented using any suitable combination of hardware and/or software. The computer-readable medium or article may include, for example, any suitable type of memory unit, memory, memory article, memory medium, storage device, storage article, storage medium and/or storage unit, for example, memory (including non-transitory memory), removable or non-removable media, erasable or non-erasable media, writeable or re-writeable media, digital or analog media, hard disk, floppy disk, Compact Disk Read Only Memory (CD-ROM), Compact Disk Recordable (CD-R), Compact Disk Rewriteable (CD-RW), optical disk, magnetic media, magneto-optical media, removable memory cards or disks, various types of Digital Versatile Disk (DVD), a tape, a cassette, or the like. The instructions may include any suitable type of code, such as source code, compiled code, interpreted code, executable code, static code, dynamic code, encrypted code, programming code, and the like, implemented using any suitable high-level, low-level, object-oriented, visual, compiled and/or interpreted programming language. The non-transitory computer readable medium embodied programming code may cause a processor when executing the programming code to perform functions, such as those described herein.

Certain examples of the present disclosure were described above. It is, however, expressly noted that the present disclosure is not limited to those examples, but rather the intention is that additions and modifications to what was expressly described herein are also included within the scope of the disclosed examples. Moreover, it is to be understood that the features of the various examples described herein were not mutually exclusive and may exist in various combinations and permutations, even if such combinations or permutations were not made express herein, without departing from the spirit and scope of the disclosed examples. In fact, variations, modifications, and other implementations of what was described herein will occur to those of ordinary skill in the art without departing from the spirit and the scope of the disclosed examples. As such, the disclosed examples are not to be defined only by the preceding illustrative description.

Program aspects of the technology may be thought of as "products" or "articles of manufacture" typically in the form of executable code and/or associated data that is carried on or embodied in a type of non-transitory, machine readable medium. Storage type media include any or all of the tangible memory of the computers, processors or the like, or associated modules thereof, such as various semiconductor memories, tape drives, disk drives and the like, which may provide non-transitory storage at any time for the software programming. It is emphasized that the Abstract of the Disclosure is provided to allow a reader to quickly ascertain the nature of the technical disclosure. It is submitted with the understanding that it will not be used to interpret or limit the scope or meaning of the claims. In addition, in the foregoing Detailed Description, various features are grouped together in a single example for streamlining the disclosure. This method of disclosure is not to be interpreted as reflecting an intention that the claimed examples require more features than are expressly recited in each claim. Rather, as the following claims reflect, inventive subject matter lies in less than all features of a single disclosed example. Thus, the following claims are hereby incorporated into the Detailed Description, with each claim standing on its own as a separate example. In the appended claims, the terms "including" and "in which" are used as the plain-English equivalents of the respective terms "comprising" and "wherein," respectively. Moreover, the terms "first," "second," "third," and so forth, are used merely as labels and are not intended to impose numerical requirements on their objects.

The foregoing description of examples has been presented for the purposes of illustration and description. It is not intended to be exhaustive or to limit the present disclosure to the precise forms disclosed. Many modifications and variations are possible in light of this disclosure. It is intended that the scope of the present disclosure be limited not by this detailed description, but rather by the claims appended hereto. Future filed applications claiming priority to this application may claim the disclosed subject matter in a different manner and may generally include any set of one or more limitations as variously disclosed or otherwise demonstrated herein.

Although the present invention is defined in the attached claims, it should be understood that the present invention can also (alternatively) be defined in accordance with the following embodiments:

1. A controller for operating a drug delivery device, comprising:

a processor; and
a memory storing instructions that, when executed by the processor, operate the controller to:

determine a plurality of dosage clusters for a patient based on drug dosage patient information,
determine an adjustment profile for each of the dosage clusters,
determine a current cluster for a dosage cycle,
determine an adjusted dosage for the dosage cycle by applying the adjustment profile to a default dosage, and
provide a signal to the drug delivery device to deliver the adjusted dosage to the patient for the dosage cycle.

2. The controller of embodiment 1, the drug delivery device comprising an automatic insulin delivery (AID) device.

3. The controller of one of the preceding embodiments, the drug comprising one of a hormone, a protein, a chemotherapy drug, a medication, glucagon, a glucagon-like peptide, or insulin.

4. The controller of one of the preceding embodiments, the drug comprising insulin.

5. The controller of one of the preceding embodiments, the instructions, when executed by the processor, to operate the controller to determine the plurality of clusters based on a time period.

6. The controller of one of the preceding embodiments, the time period comprising one of a week, a month, or a year.

7. The controller of one of the preceding embodiments, the plurality of clusters segmented based on days of the week.

8. The controller of one of the preceding embodiments, the instructions, when executed by the processor, to operate the controller to determine the plurality of clusters based on at least one cluster criterion.

9. The controller of one of the preceding embodiments, the at least one cluster criterion comprising a dosage deviation in drug dosages between portions of a time period.

10. The controller of one of the preceding embodiments, the time period comprising a month and the dosage deviation comprising a percentage difference in a dose of the drug between consecutive days of the week.

11. The controller of one of the preceding embodiments, the percentage difference comprising about 10% to about 100%.

12. The controller of one of the preceding embodiments, the instructions, when executed by the processor, to operate the controller to determine the adjustment profile for each of the dosage clusters based on an overall drug dosage mean and a cluster dosage mean.

13. The controller of one of the preceding embodiments, the instructions, when executed by the processor, to operate the controller to determine the adjustment profile for each of the dosage clusters based on (a cluster dosage mean)/(an overall drug dosage mean).

14. The controller of one of the preceding embodiments, the drug comprising insulin and the default dosage comprising a total daily insulin (TDI) amount.

15. A method for operating a drug delivery device, comprising, via a processor of a controller:

determining a plurality of dosage clusters for a patient based on drug dosage patient information;
determining an adjustment profile for each of the dosage clusters;
determining a current cluster for a dosage cycle;
determining an adjusted dosage for the dosage cycle by applying the adjustment profile to a default dosage; and
providing a signal to the drug delivery device to deliver the adjusted dosage to the patient for the dosage cycle.

16. The method of embodiment 15, the drug delivery device comprising an automatic insulin delivery (AID) device.

17. The method of one of embodiments 15 or 16, the drug comprising one of a hormone, a protein, a chemotherapy drug, a medication, glucagon, a glucagon-like peptide, or insulin.

18. The method of one of embodiments 15 to 17, the drug comprising insulin.

19. The method of one of embodiments 15 to 18, comprising determining the plurality of clusters based on a time period.

20. The method of one of embodiments 15 to19, the time period comprising one of a week, a month, or a year.

21. The method of one of embodiments 15 to 20, the plurality of clusters segmented based on days of the week.

22. The method of one of embodiments 15 to 21 comprising determining the plurality of clusters based on at least one cluster criterion.

23. The method of one of embodiments 15 to 22, the at least one cluster criterion comprising a dosage deviation in drug dosages between portions of a time period.

24. The method of one of embodiments 15 to 23, the time period comprising a month and the dosage deviation comprising a percentage difference in a dose of the drug between consecutive days of the week.

25. The method of one of embodiments 15 to 24, the percentage difference comprising about 10% to about 100%.

26. The method of one of embodiments 15 to 25, comprising determining the adjustment profile for each of the dosage clusters based on an overall drug dosage mean and a cluster dosage mean.

27. The method of one of embodiments 15 to 26, comprising determining the adjustment profile for each of the dosage clusters based on (a cluster dosage mean)/(an overall drug dosage mean).

28. The method of one of embodiments 15 to 27, the drug comprising insulin and the default dosage comprising a total daily insulin (TDI) amount.

29. A method for operating a drug delivery device to deliver a drug to a patient, the method comprising, via a processor of a controller:

  determining a response event probability for at least one response event for at least one historical time period;
  determining an aggressiveness factor based on the response event probability;
  determining a drug dosage for the drug based on the aggressiveness factor applied in a drug delivery cost function for a current time period associated with the at least one historical time period; and
  delivering the drug dosage of the drug to the patient.

30. The method of embodiment 29, the drug delivery device comprising an automatic insulin delivery (AID) device.

31. The method of one of embodiments 29 or 30, the drug comprising one of a hormone, a protein, a chemotherapy drug, a medication, glucagon, a glucagon-like peptide, or insulin.

32. The method of one of embodiments 29 to 31, the drug comprising insulin.

33. The method of one of embodiments 29 to 32, the response event comprising at least one of a hypoglycemic event or a hyperglycemic event.

34. The method of one of embodiments 29 to 33, the at least one time period comprising a delivery cycle.

35. The method of one of embodiments 29 to 34, the aggressiveness factor determined based on the following estimated aggressiveness factor R':

$$R'(i) = 9000 \cdot \left(1 + 0.2 \cdot P_{hypo}(i)\right)^2 \cdot \left(1 - 0.2 \cdot P_{hyper}(i)\right)^2,$$

where $P_{hypo}$ is the probability of a hypoglycemic response event and $P_{hyper}$ is the probability of a hyperglycemic response event.

36. The method of one of embodiments 29 to 35, the aggressiveness factor (R) determined according to the following:

$$R(i) = f(R(i-1), R(i)').$$

37. A controller for operating a drug delivery device, comprising:

a processor; and
a memory storing instructions that, when executed by the processor, operate the controller to:

determine a response event probability for at least one response event for at least one historical time period;
determine an aggressiveness factor based on the response event probability;
determine a drug dosage for the drug based on the aggressiveness factor applied in a drug delivery cost function for a current time period associated with the at least one historical time period; and
deliver the drug dosage of the drug to the patient.

38. The controller of embodiment 37, the drug delivery device comprising an automatic insulin delivery (AID) device.

39. The controller of one of embodiments 37 or 38, the drug comprising one of a hormone, a protein, a chemotherapy drug, a medication, glucagon, a glucagon-like peptide, or insulin.

40. The controller of one of embodiments 37 to 39, the drug comprising insulin.

41. The controller of one of embodiments 37 to 40, the response event comprising at least one of a hypoglycemic event or a hyperglycemic event.

42. The controller of one of embodiments 37 to 41, the at least one time period comprising a delivery cycle.

43. The controller of one of embodiments 37 to 42, the aggressiveness factor determined based on the following estimated aggressiveness factor R':

$$R'(i) = 9000 \cdot \left(1 + 0.2 \cdot P_{hypo}(i)\right)^2 \cdot \left(1 - 0.2 \cdot P_{hyper}(i)\right)^2,$$

where $P_{hypo}$ is the probability of a hypoglycemic response event and $P_{hyper}$ is the probability of a hyperglycemic response event.

44. The controller of one of embodiments 37 to 43, the aggressiveness factor (R) determined according to the following:

$$R(i) = f(R(i-1), R(i)').$$

**Claims**

1. A method for operating a drug delivery device to deliver a drug to a patient, the method comprising, via a processor of a controller:

determining a response event probability for at least one response event for at least one historical time period;
determining an aggressiveness factor based on the response event probability;
determining a drug dosage for the drug based on the aggressiveness factor applied in a drug delivery cost function for a current time period associated with the at least one historical time period; and
delivering the drug dosage of the drug to the patient.

2. The method of claim 1, the drug delivery device comprising an automatic insulin delivery (AID) device.

3. The method of one of claims 1 or 2, the drug comprising one of a hormone, a protein, a chemotherapy drug, a medication, glucagon, a glucagon-like peptide, or insulin.

4. The method of one of claims 1 to 3, the drug comprising insulin.

5. The method of one of claims 1 to 4, the response event comprising at least one of a hypoglycemic event or a hyperglycemic event.

6. The method of one of claims 1 to 5, the at least one time period comprising a delivery cycle.

7. The method of one of claims 1 to 6, the aggressiveness factor determined based on the following estimated aggressiveness factor R':

$$R'(i) = 9000 \cdot \left(1 + 0.2 \cdot P_{hypo}(i)\right)^2 \cdot \left(1 - 0.2 \cdot P_{hyper}(i)\right)^2,$$

where $P_{hypo}$ is the probability of a hypoglycemic response event and $P_{hyper}$ is the probability of a hyperglycemic response event.

8. The method of one of claims 1 to 7, the aggressiveness factor (R) determined according to the following:

$$R(i) = f(R(i-1), R(i)').$$

9. A controller for operating a drug delivery device, comprising:

a processor; and
a memory storing instructions that, when executed by the processor, operate the controller to:

determine a response event probability for at least one response event for at least one historical time period;
determine an aggressiveness factor based on the response event probability;
determine a drug dosage for the drug based on the aggressiveness factor applied in a drug delivery cost function for a current time period associated with the at least one historical time period; and
deliver the drug dosage of the drug to the patient.

10. The controller of claim 9, the drug comprising one of a hormone, a protein, a chemotherapy drug, a medication, glucagon, a glucagon-like peptide, or insulin.

11. The controller of one of claims 9 or 10, the drug comprising insulin.

12. The controller of one of claims 9 to 11, the response event comprising at least one of a hypoglycemic event or a hyperglycemic event.

13. The controller of one of claims 9 to 12, the at least one time period comprising a delivery cycle.

14. The controller of one of claims 9 to 13, the aggressiveness factor determined based on the following estimated aggressiveness factor R':

$$R'(i) = 9000 \cdot \left(1 + 0.2 \cdot P_{hypo}(i)\right)^2 \cdot \left(1 - 0.2 \cdot P_{hyper}(i)\right)^2,$$

where $P_{hypo}$ is the probability of a hypoglycemic response event and $P_{hyper}$ is the probability of a hyperglycemic response event.

15. The controller of one of claims 9 to 14, the aggressiveness factor (R) determined according to the following:

$$R(i) = f(R(i-1), R(i)').$$

*100*

Drug Delivery System *105*

Controller *110*

Processor Circuitry *120*

Memory Unit *130*

Patient Information
*132*

Profile Information
*134*

Drug Delivery Management
Application
*140*

Transceiver
*180*

User Interface
*182*

Patient
*150*

*111*

Drug Delivery Device
*160*

*112*

Analyte Sensor
*170*

Network
*190*

Data
Source
*192a*

...

Data
Source
*192n*

*FIG. 1*

201

| 220 | Sunday | Monday | Tuesday | Wednesday | Thursday | Friday | Saturday |
|---|---|---|---|---|---|---|---|
| TDI, week 1 [U] | 25 | 21 | 21 | 17 | 22 | 18 | 26 |
| TDI, week 2 [U] | 27 | 19 | 16 | 20 | 18 | 17 | 28 |
| TDI, week 3 [U] | 24 | 20 | 17 | 18 | 20 | 20 | 27 |
| TDI, week 4 [U] | 26 | 20 | 19 | 18 | 19 | 19 | 25 |

202

| 220 | Sunday | Monday | Tuesday | Wednesday | Thursday | Friday | Saturday |
|---|---|---|---|---|---|---|---|
| TDI, week 1 [U] | 25 | 21 | 21 | 17 | 22 | 18 | 26 |
| TDI, week 2 [U] | 27 | 19 | 16 | 20 | 18 | 17 | 28 |
| TDI, week 3 [U] | 24 | 20 | 17 | 18 | 20 | 20 | 27 |
| TDI, week 4 [U] | 26 | 20 | 19 | 18 | 19 | 19 | 25 |

203

| 220 | Sunday | Monday | Tuesday | Wednesday | Thursday | Friday | Saturday |
|---|---|---|---|---|---|---|---|
| TDI, week 1 [U] | 25 | 21 | 21 | 17 | 22 | 18 | 26 |
| TDI, week 2 [U] | 27 | 19 | 16 | 20 | 18 | 17 | 28 |
| TDI, week 3 [U] | 24 | 20 | 17 | 18 | 20 | 20 | 27 |
| TDI, week 4 [U] | 26 | 20 | 19 | 18 | 19 | 19 | 25 |
| Mean Delivery [U] | 25.5 | 20 | 18.25 | 18.25 | 19.75 | 18.5 | 26.5 |
| Mean Difference vs next day of week 221 | _A_ 21.6% | 8.8% | 0.0% | -8.2% | 6.3% | -43.2% | _B_ 3.8% |

FIG. 2

305

| 320 | Overall Mean TDI [U] | Cluster mean TDI [U] | Deviation, overall vs cluster mean TDI [U] | 321 Adjustment Profile |
|---|---|---|---|---|
| Cluster 1 (Days 2-6) | | 18.95 | -2.01 | -9.61% |
| Cluster 2 (Days 1,7) | 20.96 | 26 | 5.04 | 24.02% |

*FIG. 3*

_400_

```
┌─────────────────────────────────────────────┐
│   RECEIVE DRUG DOSAGE PATIENT INFORMATION     │
│                    402                        │
└─────────────────────────────────────────────┘
                      │
                      ▼
┌─────────────────────────────────────────────┐
│          DETERMINE DOSAGE CLUSTERS            │
│                    404                        │
└─────────────────────────────────────────────┘
                      │
                      ▼
┌─────────────────────────────────────────────┐
│       DETERMINE PATIENT DOSAGE PROFILE        │
│                    406                        │
└─────────────────────────────────────────────┘
                      │
                      ▼
┌─────────────────────────────────────────────┐
│   DETERMINE ADJUSTED DOSAGE FOR CURRENT       │
│                 PROFILE                       │
│                    408                        │
└─────────────────────────────────────────────┘
                      │
                      ▼
┌─────────────────────────────────────────────┐
│  DELIVER DRUG TO PATIENT USING ADJUSTED DOSAGE│
│                    410                        │
└─────────────────────────────────────────────┘
```

*FIG. 4*

_500_

```
┌─────────────────────────────────────────────┐
│           DETERMINE DEFAULT TDI               │
│                   502                         │
└─────────────────────────────────────────────┘
                      │
                      ▼
┌─────────────────────────────────────────────┐
│  DETERMINE TIME PERIOD INSULIN DELIVERY       │
│  PATTERNS                                     │
│                   504                         │
└─────────────────────────────────────────────┘
                      │
                      ▼
┌─────────────────────────────────────────────┐
│      DETERMINE INSULIN DELIVERY CLUSTERS      │
│                   506                         │
└─────────────────────────────────────────────┘
                      │
                      ▼
┌─────────────────────────────────────────────┐
│       GENERATE TDI ADJUSTMENT PROFILE         │
│                   508                         │
└─────────────────────────────────────────────┘
                      │
                      ▼
┌─────────────────────────────────────────────┐
│ DELIVER INSULIN TO PATIENT USING ADJUSTED     │
│ TDI FOR CURRENT CLUSTER                       │
│                   510                         │
└─────────────────────────────────────────────┘
```

_FIG. 5_

601

| 620 | 621 | 622 | 623 | 624 | 625 | 626 | 627 | 628 |
|---|---|---|---|---|---|---|---|---|
| time | day 1 | day 2 | day 3 | day 5 | day 6 | day 7 | Hypoglycemia Probability | Hyperglycemia Probability |
| 15:00 | 45 | 65 | 85 | 105 | 125 | 145 | 29% | 0% |
| 15:05 | 50 | 70 | 90 | 110 | 130 | 150 | 29% | 0% |
| 15:10 | 55 | 75 | 95 | 115 | 135 | 155 | 14% | 0% |
| 15:15 | 60 | 80 | 100 | 120 | 140 | 160 | 14% | 0% |
| 15:20 | 65 | 85 | 105 | 125 | 145 | 165 | 14% | 0% |
| 15:25 | 70 | 90 | 110 | 130 | 150 | 170 | 14% | 0% |
| 15:30 | 75 | 95 | 115 | 135 | 155 | 175 | 0% | 0% |
| 15:35 | 80 | 100 | 120 | 140 | 160 | 180 | 0% | 14% |
| 15:40 | 85 | 105 | 125 | 145 | 165 | 185 | 0% | 14% |
| 15:45 | 90 | 110 | 130 | 150 | 170 | 190 | 0% | 14% |
| 15:50 | 95 | 115 | 135 | 155 | 175 | 195 | 0% | 14% |
| 15:55 | 100 | 120 | 140 | 160 | 180 | 200 | 0% | 29% |

*FIG. 6*

_700_

DETERMINE DEFAULT DRUG DELIVERY COST FUNCTION
_702_

DETERMINE TIME PERIOD INSULIN DELIVERY PATTERNS
_704_

DETERMINE RESPONSE EVENT PROBABILITIES
_706_

GENERATE AGGRESSIVENESS ADJUSTMENT
_708_

DELIVER INSULIN TO PATIENT USING ADJUSTED
AGGRESSIVENESS FOR CURRENT TIME PERIOD
_710_

*FIG. 7*

**FIG. 8**

*800*

**Controller 805**

Communication Device 854
- Transceiver A 852
- Transceiver B 856

User Interface 858 | Proc 851

Mem 853
- Proc Code 857
- MDA 859

Automatic Analyte Control 816

Cloud-Based Services 811

Communication Link 815

893

894

895

**Smart Accessory Device 807**

Comm. Device 874 | Proc 871 | UI 878 | Mem 873 — MDA App 879

891

892

877

**Drug Delivery Device 802**

UI 827

PROC 821

Pump Mech. 825

Comm. Device 826

Mem 823
- AAC 814
- MDA 829

Reservoir 824

Pwr Source 828

Sensors 884

808

**Analyte Sensor 803**

Proc 841 | Mem 832 — MDA App 836

Communication Device 835

Sensing/Measuring Device 833

EP 4 141 881 A1

Europäisches
Patentamt

European
Patent Office

Office européen
des brevets

**EUROPEAN SEARCH REPORT**

Application Number

EP 22 19 2768

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/203038 A1 (DESBOROUGH LANE [US] ET AL) 20 July 2017 (2017-07-20) * paragraphs [0047], [0048], [0055], [0063], [0118]; figures 1, 6-8 * ----- | 1-15 | INV. G16H20/17 G16H20/60 G16H50/20 G16H50/70 |
| X | US 2021/187197 A1 (ZADE ASHUTOSH [US] ET AL) 24 June 2021 (2021-06-24) * paragraphs [0047] – [0063], [0077], [0078]; figure 1 * ----- | 1-15 | |

TECHNICAL FIELDS
SEARCHED      (IPC)

G16H

The present search report has been drawn up for all claims

| Place of search | Date of completion of the search | Examiner |
|---|---|---|
| Munich | 13 January 2023 | Flores Sanchez, L |

## ANNEX TO THE EUROPEAN SEARCH REPORT
## ON EUROPEAN PATENT APPLICATION NO.

EP 22 19 2768

This annex lists the patent family members relating to the patent documents cited in the above-mentioned European search report.
The members are as contained in the European Patent Office EDP file on
The European Patent Office is in no way liable for these particulars which are merely given for the purpose of information.

13-01-2023

| Patent document cited in search report | | Publication date | Patent family member(s) | | Publication date |
|---|---|---|---|---|---|
| US 2017203038 | A1 | 20-07-2017 | AU | 2017207484 A1 | 28-06-2018 |
| | | | AU | 2020294328 A1 | 18-03-2021 |
| | | | AU | 2021204350 A1 | 22-07-2021 |
| | | | AU | 2021204355 A1 | 22-07-2021 |
| | | | CA | 3009351 A1 | 20-07-2017 |
| | | | CN | 108495665 A | 04-09-2018 |
| | | | CN | 112933333 A | 11-06-2021 |
| | | | CN | 113101448 A | 13-07-2021 |
| | | | EP | 3374004 A1 | 19-09-2018 |
| | | | EP | 3443998 A1 | 20-02-2019 |
| | | | EP | 3453414 A1 | 13-03-2019 |
| | | | HK | 1257093 A1 | 11-10-2019 |
| | | | JP | 6876046 B2 | 26-05-2021 |
| | | | JP | 7171814 B2 | 15-11-2022 |
| | | | JP | 2019509074 A | 04-04-2019 |
| | | | JP | 2021129995 A | 09-09-2021 |
| | | | US | 2017203037 A1 | 20-07-2017 |
| | | | US | 2017203038 A1 | 20-07-2017 |
| | | | US | 2017203039 A1 | 20-07-2017 |
| | | | US | 2017232195 A1 | 17-08-2017 |
| | | | US | 2019247578 A1 | 15-08-2019 |
| | | | US | 2020376197 A1 | 03-12-2020 |
| | | | US | 2021030955 A1 | 04-02-2021 |
| | | | US | 2021060245 A1 | 04-03-2021 |
| | | | US | 2022031949 A1 | 03-02-2022 |
| | | | WO | 2017124006 A1 | 20-07-2017 |
| US 2021187197 | A1 | 24-06-2021 | EP | 4076154 A1 | 26-10-2022 |
| | | | US | 2021187197 A1 | 24-06-2021 |
| | | | WO | 2021126915 A1 | 24-06-2021 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7303549 B
- US 7137964 B
- US 6740059 B
- US 840483